# EUROPEAN PATENT APPLICATION

(11) **EP 4 290 636 A1**
(43) Date of publication of application: **13.12.2023**
(21) Application number: 21931129.7
(22) Date of filing: 27.08.2021
(51) Int. Cl.: H01M 10/0567, H01M 10/0525

(54) **ELECTROLYTE ADDITIVE, ELECTROLYTE, AND LITHIUM-ION BATTERY**

(30) Priority: 16.03.2021 CN 202110279447
(71) Applicant: Guangzhou Tinci Materials Technology Co., Ltd., Guangzhou, Guangdong 510760 (CN)
(72) Inventor: CAO, Gejin, Guangzhou, Guangdong 510760 (CN); FAN, Weizhen, Guangzhou, Guangdong 510760 (CN); FAN, Chaojun, Guangzhou, Guangdong 510760 (CN); XIN, Yong, Guangzhou, Guangdong 510760 (CN); ZHAO, Jingwei, Guangzhou, Guangdong 510760 (CN)
(74) Representative: Bandpay & Greuter
(86) International application number: PCT/CN2021/115026
(87) International publication number: WO 2022/193554

(57) **Abstract**

An electrolyte additive, an electrolyte, and a lithium-ion battery, where the electrolyte additive has a structure shown in formula (I): 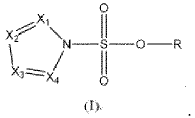 The electrolyte additive has both a film-forming property and the property of inhibiting the rise of acidity and chromaticity of the electrolyte, thereby effectively improving the cycle performance of the battery.

## Description

### TECHNICAL FIELD

The present invention relates to the technical field of batteries, and in particular to an electrolyte additive, electrolyte, and a lithium-ion battery.

### BACKGROUND

Lithium-ion battery electrolyte is generally composed of a lithium salt, a solvent and an additive. Lithium hexafluorophosphate, as a lithium salt widely used commercially at present, has advantages of good solubility and high electrical conductivity. However, lithium hexafluorophosphate has poor thermal stability and is easy to undergo a decomposition reaction (LiPF₆ → LiF + PF₅) at high temperature, producing phosphorus pentafluoride, which is chemically active and can react with trace impurities in the electrolyte, resulting in a rapid increase in acidity and chromaticity of the electrolyte, deteriorating the quality of the electrolyte and reducing the capacity and cycle performance of the battery. The commonly used additives such as vinylene carbonate (VC), fluoroethylene carbonate (FEC) and ethylene sulfate (DTD) have good film-forming effect, but have no improved effect on the stability of the electrolyte. Furthermore, DTD as film-forming additive has poor stability itself, and will further aggravate the deterioration of quality of the electrolyte at high temperature. Phosphite esters are often used as electrolyte stabilizers in the industry; although the additives help to inhibit the rise of the chromaticity of the electrolyte, they have poor compatibility with the graphite as negative electrode, and even in the case of trace addition of a few hundred ppm, they will have a slight negative effect on the cycle performance of the battery.

Therefore, there is an urgent need to develop a class of electrolyte additives that have both a film-forming property and a property of inhibiting rise of acidity and chromaticity of the electrolyte.

### SUMMARY

In view of this, it is necessary to provide an electrolyte additive, an electrolyte and a lithium-ion battery, where the electrolyte additive has both a film-forming property and a property of inhibiting rise of acidity and chromaticity of electrolyte.

An electrolyte additive has a structure shown in Formula (I): 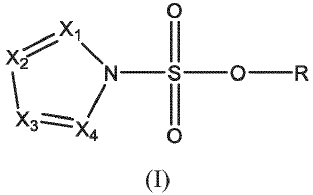 one of X₁, X₂, X₃ and X₄ is N, and the rest are CR₁;
R₁ is selected from: H, a 5-6 membered aryl, a 5-6 membered heteroaryl, a C₁₋₈ alkyl, a C₂₋₈ alkenyl, a C₀₋₈ alkylsilyl, a R₀ substituted 5-6 membered aryl, a R₀ substituted 5-6 membered heteroaryl, a R₀ substituted C₁₋₈ alkyl, a R₀ substituted C₂₋₈ alkenyl, or a R₀ substituted C₀₋₈ alkylsilyl;
R is selected from: a 5-6 membered aryl, a 5-6 membered heteroaryl, a C₁₋₈ alkyl, a C₂₋₈ alkenyl, a C₀₋₈ alkylsilyl, a R₀ substituted 5-6 membered aryl, a R₀ substituted 5-6 membered heteroaryl, a R₀ substituted C₁₋₈ alkyl, a R₀ substituted C₂₋₈ alkenyl, or a R₀ substituted C₀₋₈ alkylsilyl;
R₀ is selected from: a C₁₋₆ alkyl, a C₁₋₆ alkoxy or a halogen.

An electrolyte includes additive, the additive includes a first additive, and the first additive is the electrolyte additive as described in the above.

A lithium-ion battery includes a positive electrode material, a negative electrode material and the electrolyte as described in the above.

### The present invention has the following beneficial effects:

By using the electrolyte additive including nitrogen-containing five-membered heterocyclic sulfonate compounds with the structure shown in formula (I), it is possible to effectively inhibit the rise of acidity and chromaticity of the electrolyte, improve the stability of the electrolyte at high temperature, and these compounds have excellent film-forming property, and can be reduced at the negative electrode to form a stable SEI film during a first charging of the battery, and thus they can effectively improve the cycle performance of the battery. The skilled of the present invention speculates that the principle for producing the above technical effects is as follows:
The nitrogen-containing five-membered heterocycle in the compound shown in formula (I) contains a nitrogen atom with a lone electron pair, which makes the compound show weak Lewis basicity in the electrolyte and be capable of forming a six-ligand complex with PF₅, reducing the Lewis acidity and reactivity of PF₅, effectively inhibiting the rise of acidity of the electrolyte and the rise of the chromaticity caused by the reaction of PF₅ with a trace amount of impurities in the electrolyte. In addition, the above compounds have good compatibility with the graphite as negative electrode, and can form a film on the surface of the electrode when adding to the electrolyte. Alkylated lithium sulfate produced by the decomposition of the above compounds introduces element S into the SEI film, increasing the ionic conductivity, and thus can effectively improve the cycle performance of the lithium-ion battery.

### BRIEF DESCRIPTION OF DRAWINGS

FIG. 1 shows views of electrolytes of Comparative Example 1, Comparative Example 2, Example 4, Example 6, Example 7 and Example 8 before and after storage.
FIG. 2 is dQ/dV curve diagrams of Comparative Example 1, Comparative Example 2 and Example 4.

### DESCRIPTION OF EMBODIMENTS

In order to facilitate the understanding of the present invention, a more comprehensive description of the present invention will be given below, and preferred embodiments of the present invention are given. However, the present invention can be implemented in many different forms and is not limited to the embodiments described herein. On the contrary, the purpose of providing these embodiments is to enable a more thorough and comprehensive understanding of the disclosed content of the present invention.

Unless otherwise defined, all technical and scientific terms used herein have the same meaning as generally understood by the skilled in the field of the present invention. The terms used in the specification of the present invention are only for the purpose of describing specific embodiments and are not intended to limit the present invention. The terms "and/or" used herein include any and all combinations of one or more relevant listed items.

### Explanations of terms

The term "alkyl" refers to a saturated hydrocarbon containing a primary (positive) carbon atom, or a secondary carbon atom, or a tertiary carbon atom, or a quaternary carbon atom, or a combination thereof. A phrase including the term, for example, "C₁₋₈ alkyl" refers to an alkyl containing 1 to 8 carbon atoms. Suitable examples include, but are not limited to: methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃ and octyl (-(CH₂)₇CH₃).

"Alkenyl" refers to a hydrocarbon containing a normal, secondary, tertiary or cyclic carbon atom with at least one unsaturated site, i.e., a carbon-carbon sp² double bond. A phrase including the term, for example, "C₂₋₈ alkenyl" refers to an alkenyl group containing 2 to 8 carbon atoms. Suitable examples include, but are not limited to: vinyl (-CH=CH₂), propenyl (-CH₂CH=CH₂), cyclopentenyl (-C₅H₇) and 5-hexenyl (-CH₂CH₂CH₂CH₂CH=CH₂).

"Heteroaryl" refers to an aryl group in which at least one carbon atom is substituted with a non-carbon atom, where the non-carbon atom can be N atom, O atom, S atom and so on. Suitable examples include, but are not limited to: furan, benzofuran, thiophene, benzothiophene, pyrrole, pyrazole, triazole, imidazole, oxazole, oxadiazole, thiazole, tetrazole, indole, carbazole, pyrroloimidazole, pyrrolopyrrole, thienopyrrole, thienothiophene, furanopyrrole, furanofuran, thienofuran, benzoisoxazole, benzoisothiazole, benzimidazole, pyridine, pyrazine, pyridazine, pyrimidine, triazine, quinoline, isoquinoline, cinnoline, quinoxaline, phenanthridine, perimidine, quinazoline and quinazolinone.

"Halogen" or "halogen atom" refers to F, Cl, Br or I.

The term "halogen substituted" or "halogenated" denotes that H at any position and in any number on a corresponding group is substituted with a halogen; for example, fluoromethyl, including monofluoromethyl, difluoromethyl, trifluoromethyl; for example, fluoroethyl including but is not limited to, CH₃CH₂F, CH₂FCH₂F, CF₂HCH₃, CF₃CH₃, CF₃CF₃, etc.

"Silicon group" or "silyl" refers to 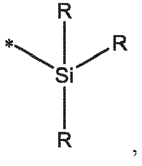 in which R may be an acceptable group in the field, such as C₁₋₈ alkyl (preferably C₁₋₆ alkyl, more preferably C₁₋₄ alkyl), H or halogen (preferably F); and in which a plurality of R may be the same or different from each other.

"C₀₋₈ alkylsilyl" refers to 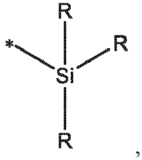 in which R is C₀₋₈ alkyl, and it can be understood that when R is C₀ alkyl, it means that in 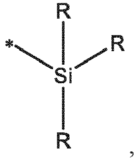 R contains no carbon atom, that is, all R is H, equivalent to 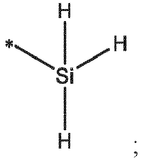 "halogen substituted C₀₋₈ alkylsilyl" refers to that in 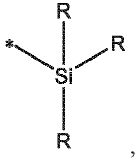 R is halogen substituted C₀₋₈ alkyl, and when R is C₀ alkyl, it means that in 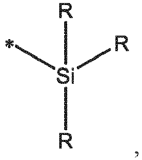 R contains no carbon atom, R is H or halogen, and at least one R is halogen, such as fluorine-substituted C₀ alkylsilyl, equivalent to

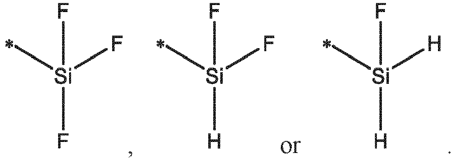

### Detailed explanation

An embodiment of the present invention provides an electrolyte additive with a structure shown in Formula (I): 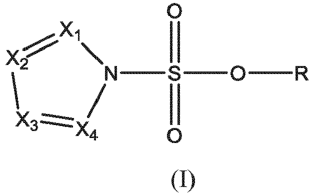 one of X₁, X₂, X₃ and X₄ is N, and the rest are CR₁;
R₁ is selected from: H, a 5-6 membered aryl, a 5-6 membered heteroaryl, a C₁₋₈ alkyl, C₂₋₈ alkenyl, a C₀₋₈ alkylsilyl, a R₀ substituted 5-6 membered aryl, a R₀ substituted 5-6 membered heteroaryl, a R₀ substituted C₁₋₈ alkyl, a R₀ substituted C₂₋₈ alkenyl, or a R₀ substituted C₀₋₈ alkylsilyl;
R is selected from: a 5-6 membered aryl, a 5-6 membered heteroaryl, a C₁₋₈ alkyl, a C₂₋₈ alkenyl, a C₀₋₈ alkylsilyl, a R₀ substituted 5-6 membered aryl, a R₀ substituted 5-6 membered heteroaryl, a R₀ substituted C₁₋₈ alkyl, a R₀ substituted C₂₋₈ alkenyl, or a R₀ substituted C₀₋₈ alkylsilyl;
R₀ is selected from: a C₁₋₆ alkyl, C₁₋₆ alkoxy or a halogen.

In one example, X₃ is N; X₁, X₂ and X₄ are CR₁.

In one example, X₃ is N; X₁, X₂ and X₄ are CH, that is, they are selected from a structure shown in the following general formula:

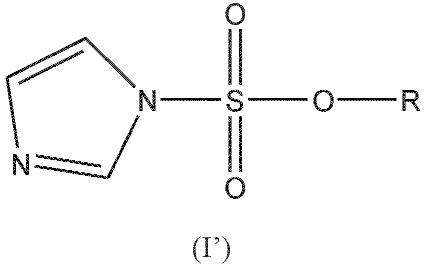

In one example, R₁ is selected from: H, a C₁₋₆ alkyl, a C₂₋₆ alkenyl, a R₀ substituted C₁₋₆ alkyl or a R₀ substituted C₂₋₆ alkenyl.

In one example, R is selected from: a 5-6 membered aryl, a 5-6 membered heteroaryl, a C₁₋₆ alkyl, a C₂₋₆ alkenyl, a C₀₋₆ alkylsilyl, a R₀ substituted 5-6 membered aryl, a R₀ substituted 5-6 membered heteroaryl, a R₀ substituted C₁₋₆ alkyl, a R₀ substituted C₂₋₆ alkenyl, or a R₀ substituted C₀₋₆ alkylsilyl.

In one example, the 5-6 membered heteroaryl is selected from: furyl, thienyl, pyrrolyl, pyrazolyl, triazolyl, thiazolyl, imidazolyl, pyridyl, pyrazinyl, pyrimidinyl, pyridazinyl or triazinyl.

In one example, R₀ is selected from: a C₁₋₄ alkyl or a halogen; further, R₀ is selected from a halogen, and further R₀ is selected from fluorine.

In one example, R is selected from: phenyl, thienyl, imidazolyl, pyridyl, fluorophenyl, fluorothienyl, fluorimidazolyl, fluoropyridine, methyl, ethyl, 1-propyl, 2-propyl, 1-butyl, 2-methyl-1-propyl, 2-butyl, fluoromethyl, fluoroethyl, fluoro-1-propyl, fluoro-2-propyl, fluoro-1-butyl, fluoro-2-methyl-1-propyl, fluoro-2-butyl, vinyl, propenyl, butenyl, fluorovinyl, fluoropropenyl, fluorobutenyl, trimethylsilyl, triethylsilyl, trifluorosilyl, tri(trifluoromethyl)dimethylsilyl, di(trifluoromethyl)methylsilyl, or tris(trifluoromethyl)silyl.

In one example, R is selected from: phenyl, fluorophenyl, imidazolyl, methyl, vinyl, trifluoromethyl or trimethylsilyl.

In one example, is selected from any one of the following compounds:

| | |
|---|---|
| 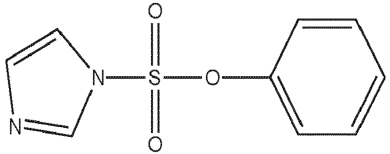 | 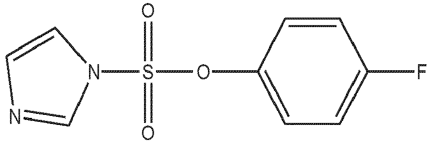 |
| (I1) | (I2) |
| 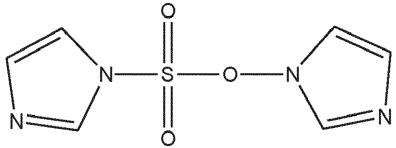 | 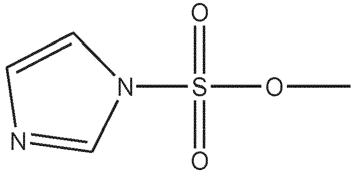 |
| (I3) | (I4) |
| 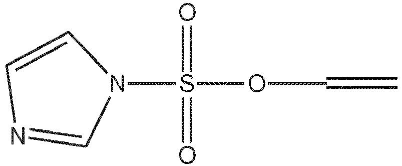 | 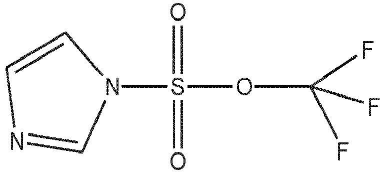 |
| (I5) | (I6) |
| 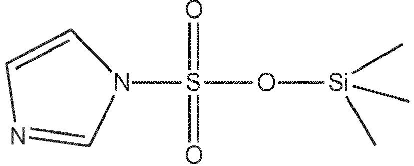 | |
| (I7) | |

For the preparation of the above electrolyte additives, please refer to CN105121404A.

An embodiment of the present invention provides use of a nitrogen-containing five-membered heterocyclic sulfonate with the structure shown in formula (I) as an electrolyte additive: 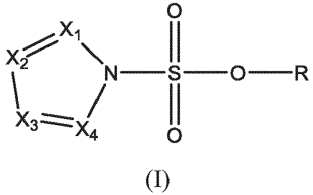 in which, groups are as defined above, and will not be repeated here.

An embodiment of the present invention provides use of a nitrogen-containing five-membered heterocyclic sulfonate shown in formula (I) in preparation of an electrolyte: 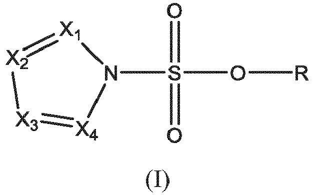 in which, groups are as defined above, and will not be repeated here.

An embodiment of the present invention provides an electrolyte, including an additive, where the additive includes a first additive, and the first additive is the electrolyte additive as described above.

In one example, the above additive further includes a second additive, the second additive is selected from at least one of vinylene carbonate, fluoroethylene carbonate, 1,3-propylene sulfonic lactone, 1,3-propane sulfonic lactone, ethylene sulfate or methylene methanedi sulfonate.

In one example, the electrolyte is a non-aqueous electrolyte.

In one example, a content of the additive in the electrolyte is 0.01% to 30% by mass percent, further the content of electrolyte additive is 0.01% to 10% by mass percent; in one example, the above electrolyte further includes a lithium salt and a solvent; in one example, a content of the lithium salt in the electrolyte is 5% to 20% by mass percent and a content of the solvent is 50% to 94.9% by mass percent.

In one example, a content of the first additive in the electrolyte is 0.01% to 10% by mass percent; further the content of the first additive is 0.1% to 10% by mass percent.

In one example, a content of the second additive in the electrolyte is 0.01% to 5% by mass percent.

If the content of the first additive is too low, a SEI film formed in the negative electrode is incomplete, and the effect of improving the subsequent cycle of the battery is not good; if the content of the first additive is too high, the ability to reduction to form the film is too strong, and the SEI film formed on the surface of the negative electrode is too thick, which will increase an internal resistance of the battery, and thus have a negative effect on the performance of the battery. Therefore, by controlling the content of the additive in the above range, the stability and the film-forming effect of the electrolyte can be ensured, thereby improving the cycle performance of the battery.

In one example, in the above electrolyte, the lithium salt is selected from at least one of lithium hexafluorophosphate, lithium tetrafluoroborate, lithium bis(oxalate)borate, lithium difluorophosphate, lithium difluoroxalate phosphate, and lithium bis(fluorosulfonyl)imide.

In one example, in the above electrolyte, the solvent includes a cyclic solvent and/or a linear solvent; where the cyclic solvent is selected from at least one of ethylene carbonate, propylene carbonate, γ-butyrolactone, phenyl acetate, 1,4-butyl sultone and propylene 3,3,3-trifluorocarbonate; the linear solvent is selected from at least one of dimethyl carbonate, methyl ethyl carbonate, diethyl carbonate, ethyl acetate, methyl propyl carbonate, propyl propionate, 1,1,2,2-tetrafluoroethyl-2,2,3,3-tetrafluoropropyl ether, 2,2-difluoroethyl acetate, 2,2-difluoroethyl propionate and methyl 2,2-difluoroethyl carbonate.

An embodiment of the present prevention provides an energy storage device, including the above electrolyte, which is specifically as described above and will not be repeated here. In one example, the energy storage device is a lithium-ion battery.

In an example, a positive electrode material of the lithium-ion battery includes one or more of Li₁₊ₐ(NiₓCo_{y}M_{1-x-y})O₂, Li(NiₚMn_{q}Co_{2-p-q})O₄ and LiMₕ(PO₄)ₘ, where 0≤a≤0.3, 0≤x≤1, 0≤y≤1, 0<x+y≤1; 0≤p≤2, 0≤q≤2, 0<p+q≤2; 0<h<5, 0<m<5; M is Fe, Ni, Co, Mn, Al or V.

In an example, a negative electrode material of the lithium-ion battery includes at least one of a metal lithium, a lithium alloy, a carbon, a silicon-based negative electrode material and a tin-based negative electrode material.

The above lithium-ion battery can effectively inhibit the rise of acidity and chromaticity of the electrolyte by using the electrolyte including the nitrogen-containing five-membered heterocyclic sulfonate compounds, and the above electrolyte additive has good compatibility with graphite as the negative electrode and they can react on a surface of the electrode to form a film. The alkylated lithium sulfate produced by the decomposition of the electrolyte additive introduces element S into the SEI film, increasing the ionic conductivity and improving the cycle performance of the lithium-ion battery.

Specific examples are listed below to illustrate the present invention, but the present invention is not limited to the following examples. In the following examples, the reagents, materials and instruments used can be commercially available unless otherwise specified.

### Example 1

### (1) Composition of electrolyte:

A structure of an electrolyte additive in the present example is as shown in formula (I1).

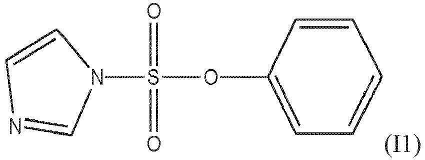

In the present example, the compound shown in formula (I1) accounts for 0.3% of a weight of the electrolyte; a conventional additive is VC, and VC accounts for 1% of the weight of the electrolyte, a lithium salt is lithium hexafluorophosphate, and the lithium salt accounts for 13% of the weight of the electrolyte; a solvent is a mixture of ethylene carbonate and methyl ethyl carbonate at a weight ratio of 1:2; and the electrolyte of Example 1 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 1. They were assembled into a soft package battery according to a conventional method.

### Example 2

### (1) Composition of electrolyte:

A structure of an electrolyte additive in the present example is shown in formula (I3).

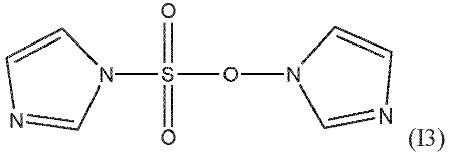

In the present example, the compound shown in formula (I3) accounts for 0.3% of a weight of the electrolyte; a conventional additive is VC and VC accounts for 1% of the weight of the electrolyte; a lithium salt is lithium hexafluorophosphate, and the lithium salt accounts for 13% of the weight of the electrolyte; a solvent is a mixture of ethylene carbonate and methyl ethyl carbonate at a weight ratio of 1:2; and the electrolyte of Example 2 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 2. They were assembled into a soft package battery according to a conventional method.

### Example 3

### (1) Composition of electrolyte:

A structure of an electrolyte additive in the present example is shown in formula (I5).

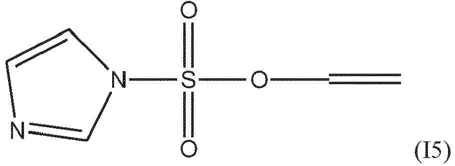

In the present example, the compound shown in formula (I5) accounts for 0.3% of a weight of the electrolyte; a conventional additive is VC and VC accounts for 1% of the weight of the electrolyte; a lithium salt is lithium hexafluorophosphate, and the lithium salt accounts for 13% of the weight of the electrolyte; a solvent is a mixture of ethylene carbonate and methyl ethyl carbonate at a weight ratio of 1:2; and the electrolyte of Example 3 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 3. They were assembled into a soft package battery according to a conventional method.

### Example 4

### (1) Composition of electrolyte:

A structure of an electrolyte additive in the present example is shown in formula (I1).

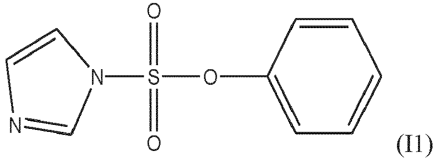

In the present example, the compound shown in formula (I1) accounts for 0.5% of a weight of the electrolyte; a conventional additive is VC and VC accounts for 1% of the weight of the electrolyte, a lithium salt is lithium hexafluorophosphate, and the lithium salt accounts for 13% of the weight of the electrolyte; a solvent is a mixture of ethylene carbonate and methyl ethyl carbonate at a weight ratio of 1:2; and the electrolyte of Example 4 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 4. They were assembled into a soft package battery according to a conventional method.

### Example 5

### (1) Composition of electrolyte:

A structure of an electrolyte additive in the present example is shown in formula (I1).

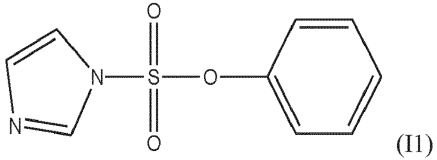

In the present example, the compound shown in formula (I1) accounts for 10% of a weight of the electrolyte; a conventional additive is VC and VC accounts for 1% of the weight of the electrolyte, a lithium salt is lithium hexafluorophosphate, and the lithium salt accounts for 13% of the weight of the electrolyte; a solvent is a mixture of ethylene carbonate and methyl ethyl carbonate at a weight ratio of 1:2; and the electrolyte of Example 5 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 5. They were assembled into a soft package battery according to a conventional method.

### Example 6

### (1) Composition of electrolyte:

A structure of an electrolyte additive in the present example is shown in formula (I1).

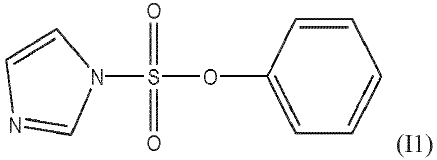

In the present example, the compound shown in formula (I1) accounts for 0.5% of a weight of the electrolyte; a conventional additive is VC and VC accounts for 1% of the weight of the electrolyte, a lithium salt is lithium hexafluorophosphate, and the lithium salt accounts for 13% of the weight of the electrolyte; a solvent is a mixture of ethylene carbonate, methyl ethyl carbonate and diethyl carbonate at a weight ratio of 3:5:2; and the electrolyte of Example 6 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 6. They were assembled into a soft package battery according to a conventional method.

### Example 7

### (1) Composition of electrolyte:

A structure of an electrolyte additive in the present example is shown in formula (I1).

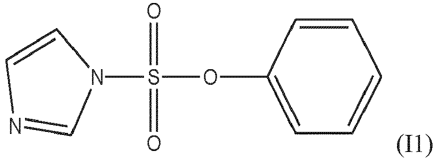

In the present example, the compound shown in formula (I1) accounts for 0.5% of a weight of the electrolyte; a conventional additive is VC and DTD, where VC and DTD each account for 1% of the weight of the electrolyte, a lithium salt is lithium hexafluorophosphate, and the lithium salt accounts for 13% of the weight of the electrolyte; a solvent is a mixture of ethylene carbonate and methyl ethyl carbonate at a weight ratio of 1:2; and the electrolyte of Example 7 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is polyethylene film; and an electrolyte is the electrolyte in Example 7. They were assembled into a soft package battery according to a conventional method.

### Example 8

### (1) Composition of electrolyte:

A structure of an electrolyte additive in the present example is shown in formula (I1).

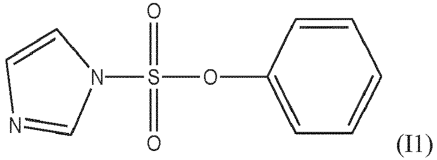

In the present example, the compound shown in formula (I1) accounts for 0.5% of a weight of the electrolyte; a conventional additive is VC and DTD, where VC and DTD each account for 1% of the weight of the electrolyte, a lithium salt is lithium hexafluorophosphate and lithium bis(fluorosulfonyl)imide, where the lithium salt and lithium bis(fluorosulfonyl)imide account for 12% and 1% of the weight of the electrolyte, respectively; a solvent is a mixture of ethylene carbonate and methyl ethyl carbonate at a weight ratio of 1:2; and the electrolyte of Example 8 was prepared according to a conventional electrolyte preparation method.

### (2) Assembling of lithium-ion battery:

A positive electrode material is LiNi_{0.8}Co_{0.1}Mn_{0.1}O₂; a negative electrode material is an artificial graphite; a separator is a polyethylene film; and an electrolyte is the electrolyte in Example 8. They were assembled into a soft package battery according to a conventional method.

### Comparative Example 1

Compared with Example 1, a difference of Comparative Example 1 is that the electrolyte does not include a sulfonate compound.

### Comparative Example 2

Compared with Example 1, a difference of Comparative Example 2 is that the electrolyte additive in Example 1 is replaced with an ethylene sulfate additive that accounts for 1% of the weight of the electrolyte.

### Comparative Example 3

Compared with Example 1, a difference of Comparative Example 3 is that the electrolyte additive of Example 1 is replaced with a triphenyl phosphite additive that accounts for 0.05% of the weight of the electrolyte.

### Comparative Example 4

Compared with Example 7, a difference of Comparative Example 6 is that the electrolyte additive of Example 7 is replaced with a triphenyl phosphite additive that accounts for 0.05% of the weight of the electrolyte.

### Comparative Example 5

Compared with Example 4, a difference of Comparative Example 5 is that the electrolyte additive of Example 4 is replaced with a phenyl benzenesulfonate additive that accounts for 0.5% of the weight of the electrolyte.

### Electrolyte stability and high-temperature performance tests of lithium-ion battery

The electrolyte stability and high-temperature performance of the lithium-ion batteries in Examples 1 to 8 and Comparative Examples 1 to 5 were tested. The tests method are as follows:
**Electrolyte stability test:** the electrolytes of the lithium-ion batteries as prepared in the above Examples 1 to 8 and Comparative Examples 1 to 5 were each charged in imported sealed aluminum bottles, which were vacuum encapsulated with an aluminum-plastic film. The electrolyte samples were simultaneously placed in a thermostat at a set temperature of 45 °C for storage. The samples were taken from a glove box to test the acidity and chromaticity of the electrolytes before storage and after 7 days, 14 days, 30 days and 60 days of storage, respectively. The acidity was tested by a potentiometric titrator with acidity value converted to HF in ppm, and the chromaticity was tested by platinum-cobalt colorimetry in Hazen.

The test results are shown in Tables 1 and 2:

**Table 1 (acidity/ppm)**

| Number | Before storage | 7 days | 14 days | 30 days | 60 days |
|---|---|---|---|---|---|
| Example 1 | 5.5 | 20.3 | 25.2 | 29.8 | 33.3 |
| Example 2 | 5.4 | 19.9 | 23.4 | 30.2 | 35.5 |
| Example 3 | 6.7 | 22.7 | 28.9 | 32.1 | 36.0 |
| Example 4 | 5.4 | 19.8 | 24.3 | 26.9 | 31.1 |
| Example 5 | 4.4 | 16.7 | 20.3 | 25.8 | 28.4 |
| Example 6 | 7.3 | 21.3 | 25.6 | 27.4 | 31.9 |
| Example 7 | 7.5 | 24.4 | 30.5 | 38.9 | 46.3 |
| Example 8 | 6.8 | 23.1 | 27.9 | 35.0 | 42.4 |
| Comparative Example 1 | 14.4 | 32.9 | 36.6 | 38.0 | 45.4 |
| Comparative Example 2 | 15.1 | 97.5 | 111.8 | 126.3 | 139.0 |
| Comparative Example 3 | 13.9 | 28.5 | 30.2 | 32.5 | 39.0 |
| Comparative Example 4 | 14.6 | 31.6 | 41.2 | 58.6 | 86.3 |
| Comparative Example 5 | 14.1 | 33.2 | 35.4 | 39.3 | 46.7 |

**Table 2 (chrominance/Hazen)**

| Number | Before storage | 7 days | 14 days | 30 days | 60 days |
|---|---|---|---|---|---|
| Example 1 | 10 | 10 | 15 | 35 | 50 |
| Example 2 | 10 | 10 | 20 | 40 | 50 |
| Example 3 | 10 | 15 | 25 | 40 | 60 |
| Example 4 | 10 | 10 | 10 | 20 | 45 |
| Example 5 | 10 | 10 | 10 | 20 | 40 |
| Example 6 | 10 | 10 | 10 | 20 | 45 |
| Example 7 | 10 | 20 | 30 | 40 | 60 |
| Example 8 | 10 | 10 | 20 | 40 | 60 |
| Comparative Example 1 | 10 | 20 | 40 | 70 | 90 |
| Comparative Example 2 | 10 | 50 | 80 | 150 | 300 |
| Comparative Example 3 | 10 | 10 | 20 | 40 | 70 |
| Comparative Example 4 | 10 | 20 | 40 | 50 | 100 |
| Comparative Example 5 | 10 | 20 | 50 | 80 | 100 |

**High-temperature cycle performance test of the battery:** the lithium-ion batteries prepared by the above Examples 1 to 8 and Comparative Examples 1 to 5 were placed in a thermostat at 45 °C, charged to 4.2 V at a constant current of 1 C and a constant voltage, and then discharged to 3.0 V at a constant current of 1 C. The capacity retention rate of the lithium-ion batteries was measured after cycling for 700 cycles.

The test results are shown in Table 3:

**Table 3**

| Number | Capacity retention rate after cycling at 45 °C for 700 cycles |
|---|---|
| Example 1 | 90.5% |
| Example 2 | 90.1% |
| Example 3 | 90.6% |
| Example 4 | 91.7% |
| Example 5 | 89.9% |
| Example 6 | 91.6% |
| Example 7 | 92.5% |
| Example 8 | 93.3% |
| Comparative Example 1 | 86.6% |
| Comparative Example 2 | 89.5% |
| Comparative Example 3 | 86.1% |
| Comparative Example 4 | 89.4% |
| Comparative Example 5 | 88.9% |

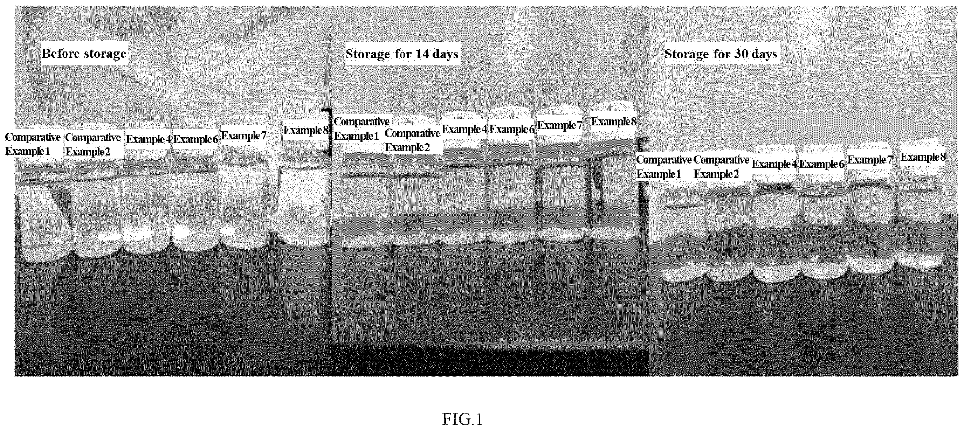

It can be known from Tables 1 to 2 that the acidity and chromaticity of the electrolytes of the lithium-ion batteries in Examples 1 to 8 are lower than those of Comparative Examples 1 to 5 when stored at 45 °C high temperature for 60 days. FIG. 1 shows views of the electrolytes of Comparative Example 1, Comparative Example 2 and Examples 4 to 8 before and after storage. It can be seen from FIG. 1 that the colors of Examples 4 to 8 are basically unchanged, but the colors of Comparative Examples 1 and 2 are significantly deepened, especially Comparative Example 2. It shows that the electrolyte additives in Examples 1 to 8 can effectively inhibit the rise of acidity and chromaticity of the electrolyte, and the electrolyte containing the electrolyte additive has enhanced stability at an elevated temperature.

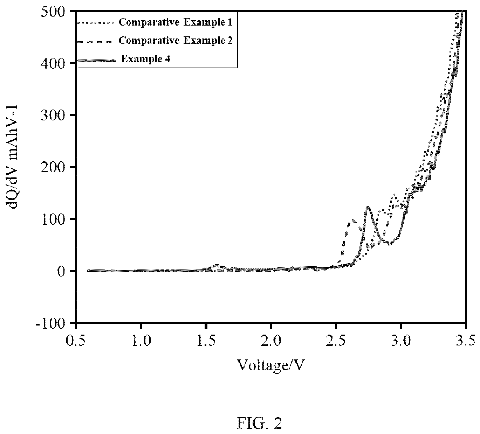

It can be known from Table 3, the high-temperature cycle performance of the lithium-ion batteries in Examples 1 to 8 is superior to that of Comparative Examples 1 to 5. FIG. 2 is dQ/dV curve diagrams of Comparative Example 1, Comparative Example 2 and Example 4. It can be seen from FIG. 2 that the cycle performance of Example 4 is significantly superior to that of Comparative Examples 1 and 2. It shows that the electrolyte additives in Examples 1 to 8 can form a stable SEI film on the surface of the negative electrode, and thus improve the high-temperature cycle performance of the lithium-ion battery.

The technical features of the above examples can be arbitrarily combined, and for the sake of brevity of description, not all possible combinations of various technical features in the above examples have been described; however, as long as there is no contradiction in the combinations of these technical features, they shall be considered to be within the scope of the present specification.

The above examples only describe several embodiments of the present invention, and are described in a more specific and detailed manner, but they cannot be understood as a limitation on the patent scope of the present invention. It should be pointed out that for a person of ordinary skill in the art, a number of deformations and improvements can be made without departing from the conception of the present invention, and they fall within the scope of protection of the present invention. Therefore, the scope of protection of the present invention patent shall be subject to the appended claims.

## Claims

1. An electrolyte additive, having a structure shown in Formula (I): 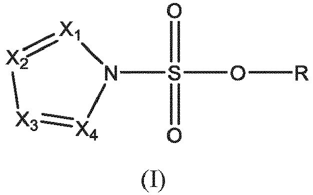 one of X₁, X₂, X₃ and X₄ is N, and the rest are CR₁;
R₁ is selected from: H, a 5-6 membered aryl, a 5-6 membered heteroaryl, a C₁₋₈ alkyl, a C₂₋₈ alkenyl, a C₀₋₈ alkylsilyl, a R₀ substituted 5-6 membered aryl, a R₀ substituted 5-6 membered heteroaryl, a R₀ substituted C₁₋₈ alkyl, a R₀ substituted C₂₋₈ alkenyl, or a R₀ substituted C₀₋₈ alkylsilyl;
R is selected from: a 5-6 membered aryl, a 5-6 membered heteroaryl, a C₁₋₈ alkyl, a C₂₋₈ alkenyl, a C₀₋₈ alkylsilyl, a R₀ substituted 5-6 membered aryl, a R₀ substituted 5-6 membered heteroaryl, a R₀ substituted C₁₋₈ alkyl, a R₀ substituted C₂₋₈ alkenyl, or a R₀ substituted C₀₋₈ alkylsilyl;
R₀ is selected from: a C₁₋₆ alkyl, a C₁₋₆ alkoxy or a halogen.

2. The electrolyte additive according to claim 1, wherein X₃ is N, and X₁, X₂ and X₄ are CH.

3. The electrolyte additive according to claim 1, wherein the 5-6 membered heteroaryl is selected from: furyl, thienyl, pyrrolyl, pyrazolyl, triazolyl, thiazolyl, imidazolyl, pyridyl, pyrazinyl, pyrimidinyl, pyridazinyl or triazinyl;
R₀ is selected from: a C₁₋₄ alkyl or a halogen.

4. The electrolyte additive according to claim 1, wherein R is selected from: phenyl, thienyl, imidazolyl, pyridyl, fluorophenyl, fluorothienyl, fluorimidazolyl, fluoropyridine, methyl, ethyl, 1-propyl, 2-propyl, 1-butyl, 2-methyl-1-propyl, 2-butyl, fluoromethyl, fluoroethyl, fluoro-1-propyl, fluoro-2-propyl, fluoro-1-butyl, fluoro-2-methyl-1-propyl, fluoro-2-butyl, vinyl, propenyl, butenyl, fluorovinyl, fluoropropenyl, fluorobutenyl, trimethylsilyl, triethylsilyl, trifluorosilyl, tri(trifluoromethyl)dimethylsilyl, di(trifluoromethyl)methylsilyl, or tris(trifluoromethyl)silyl.

5. The electrolyte additive according to claim 1, wherein the electrolyte additive is selected from any one of the following compounds:

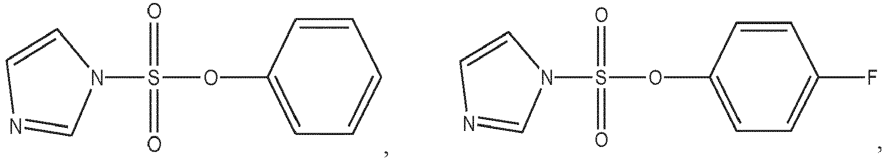

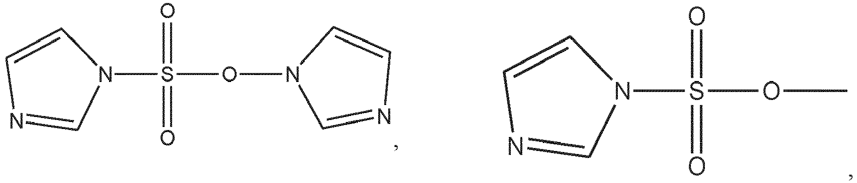

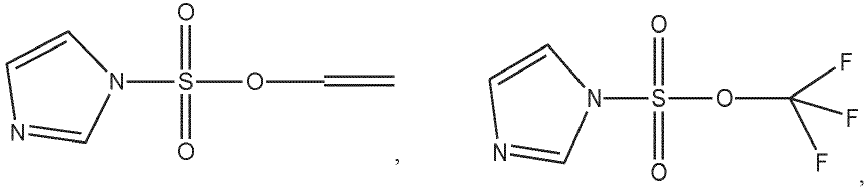

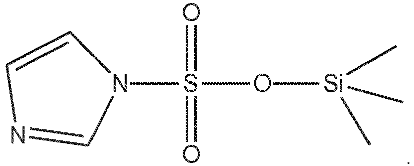

6. An electrolyte, comprising an additive, wherein the additive comprises a first additive, the first additive is the electrolyte additive according to any one of claims 1-5.

7. The electrolyte additive according to claim 6, wherein the additive further comprises a second additive, the second additive is selected from at least one of vinylene carbonate, fluoroethylene carbonate, 1,3-propylene sulfonic lactone, 1,3-propane sulfonic lactone, ethylene sulfate and methylene methanedisulfonate.

8. The electrolyte according to claim 6 or 7, further comprising a lithium salt and a solvent, wherein in the electrolyte, the additive is 0.01% to 30%, the lithium salt is 5% to 20% and the solvent is 50% to 94.9%, by mass percent.

9. The electrolyte according to claim 8, wherein the lithium salt is selected from at least one of lithium hexafluorophosphate, lithium tetrafluoroborate, lithium bis(oxalate)borate, lithium difluorophosphate, lithium difluoroxalate phosphate and lithium bis(fluorosulfonyl)imide, and/or
the solvent comprises a cyclic solvent and/or a linear solvent, wherein the cyclic solvent is selected from at least one of ethylene carbonate, propylene carbonate, γ-butyrolactone, phenyl acetate, 1,4-butyl sultone and propylene 3,3,3-trifluorocarbonate; and the linear solvent is selected from at least one of dimethyl carbonate, methyl ethyl carbonate, diethyl carbonate, ethyl acetate, methyl propyl carbonate, propyl propionate, 1,1,2,2-tetrafluorothyl-2,2,3,3-tetrafluoropropyl ether, 2,2-difluoroethyl acetate, 2,2-difluoroethyl propionate and methyl 2,2-difluoroethyl carbonate.

10. A lithium-ion battery, comprising a positive electrode material, a negative electrode material and the electrolyte according to any one of claims 4-9.
